# EUROPÄISCHE PATENTSCHRIFT

(11) **EP 1 385 490 B1**
(45) Veröffentlichungstag und Bekanntmachung des Hinweises auf die Patenterteilung: **10.06.2009**
(21) Anmeldenummer: 02727512.2
(22) Anmeldetag: 30.03.2002
(51) Int. Cl.: A61K 9/70, C09J 123/08

(54) **HAFTKLEBER AUF BASIS VON ETHYLEN-VINYLACETAT-COPOLYMEREN UND KLEBHARZEN, FÜR MEDIZINISCHE VERWENDUNGSZWECKE**
ADHESIVE EMULSION FOR MEDICAL PURPOSES MADE FROM ETHYLENE-VINYL ACETATE COPOLYMERS AND ADHESIVE RESINS
BANDE ADHESIVE A DES FINS MEDICALES A BASE DE COPOLYMERES ETHYLENE/VINYL-ACETATE ET DE RESINES ADHESIVES

(30) Priorität: 12.04.2001 DE 10118282
(43) Veröffentlichungstag der Anmeldung: 04.02.2004
(73) Patentinhaber: LTS LOHMANN Therapie-Systeme AG, 56626 Andernach (DE)
(72) Erfinder: KOCH, Andreas, 56581 Melsbach (DE); SCHMITZ, Christoph, 56598 Rheinbrohl (DE)
(74) Vertreter: Flaccus, Rolf-Dieter
(86) Internationale Anmeldenummer: PCT/EP2002/003584
(87) Internationale Veröffentlichungsnummer: WO 2002/083107

(56) Entgegenhaltungen:
- EP-A- 0 279 982
- EP-A- 0 305 756
- EP-A- 0 379 045
- GB-A- 2 037 659
- US-A- 4 814 168

## Beschreibung

Die vorliegende Erfindung betrifft Haftkleber, die auf der Basis von Ethylen-Vinylacetat-Copolymeren und Klebharzen hergestellt werden, und die sich für medizinische Verwendungszwecke eignen, insbesondere als Bestandteil von haftklebenden Hautkontaktschichten. Die Erfindung betrifft ferner Haftkleberschichten und wirkstoffhaltige haftklebende Matrixschichten, sowie transdermale therapeutische Systeme, welche derartige Haftkleber enthalten. Außerdem umfaßt die Erfindung Verfahren zur Herstellung solcher Haftkleber sowie von haftklebenden Schichten und transdermalen therapeutischen Systemen unter Verwendung der erfindungsgemäßen Haftkleber.

Druckempfindliche Haftkleber (pressure sensitive adhesives / PSA; im folgenden kurz "Haftkleber" genannt) werden im medizinischen Bereich vielfach zur Herstellung von selbstklebenden Hautkontaktschichten verwendet, beispielsweise bei Wundpflastern oder transdermalen therapeutischen Systemen (TTS). Solche Haftkleber können allgemein als hochviskose Flüssigkeiten angesehen werden, die nach einem kurzen, leichten Anpreßdruck auf die Haut sofort und dauerhaft auf dieser kleben. Aufgrund ihrer Viskoelastizität können sich solche haftklebenden Schichten sehr gut an die Hautoberfläche der unterschiedlichen Körperzonen anpassen. Die zur Zeit am häufigsten eingesetzten Haftkleber basieren in der Regel auf synthetischen Kautschukpolymeren, Polyacrylaten oder Silikonen.

Bei transdermalen therapeutischen Systemen dienen Haftkleber-Schichten häufig zugleich als Wirkstoff-Reservoir. In diesem Fall muß der Haftkleber neben seiner Klebfunktion zusätzlich die Funktion als Wirkstoffreservoir und zugleich die Kontrolle der Wirkstofffreisetzung übernehmen ("matrixkontrolliertes TTS").
Hinsichtlich der Reservoirfunktion ist zu beachten, daß der verwendete Haftkleber zu den jeweils verwendeten Wirkstoffen kompatibel ist, insbesondere darf der Haftkleber nicht zu einem Abbau oder einer Zersetzung des Wirkstoffs führen, und die chemischen Wechselwirkungen des Haftklebers mit dem Wirkstoff sollen möglichst gering sein. Außerdem wird gefordert, daß die Klebkraft des Wirkstoffreservoirs auch bei Einarbeitung größerer Mengen an Wirkstoff nicht beeinträchtigt wird.

Betrachtet man die bekannten Zusammensetzungen der Haftklebertypen kommerziell erhältlicher, "matrix-kontrollierter" TTS, so scheinen Haftkleber auf der Basis von Polyacrylaten die oben genannten Anforderungen am besten zu erfüllen. Neben ihrer sehr guten Autoadhäsion weisen Polyacrylate, im Gegensatz zu Silikonen und synthetischen Kautschukpolymeren, eine hohe Beladungskapazität auf. Von Vorteil ist außerdem das relativ geringe Hautreizungspotential von haftklebenden Schichten auf Polyacrylat-Basis.
Allerdings kann sich bei Polyacrylat-Haftklebern die Polarität oder das Vorhandensein von funktionellen Gruppen im Polymergerüst nachteilig auswirken; insbesondere im Fall von basischen, aber auch Hydrolyse-instabilen Wirkstoff-Verbindungen kann es deshalb infolge von chemischen Wechselwirkungen zu Stabilitäts- und/oder Freisetzungsproblemen kommen.
Ein weiteres Problem bei der Verwendung von Polyacrylaten als haftklebende Wirkstoffmatrix ist der oftmals notwendige Zusatz von chemischen Vernetzern, um den "kalten Fluß" (seitliches Auseinanderfließen der Wirkstoffmatrix und Austreten der Haftklebermasse an den seitlichen Rändern der gestanzten TTS während der Lagerung, aufgrund zu geringer Kohäsion im Haftkleber) zu verhindern. Allerdings sind die zugesetzten Vernetzer oft Verursacher von Stabilitätsproblemen aufgrund von chemischen Wechselwirkungen mit den im Wirkstoffreservoirs des TTS enthaltenen Wirkstoffen; falls titanhaltige Vernetzer verwendet werden, kann es zudem zu Hautirritationen kommen.

US-A-4814168 beschreibt dermale Zusammensetzungen, die einen Wirkstoff, ein Multipolymer aus Vinylacetat, Polyethylen und wahlweise einem oder mehreren Monomeren, sowie einen natürlichen oder synthetischen Kautschuk und einen Klebrigmacher enthalten. Um die Löslichkeit des Wirkstoffs in dem Polymer zu verbessern, können Cosolventien wie z. B. Lecithin, Retinolderivate, Tocopherol, Fettsäuren, Mineralöl, etc. hinzugefügt werden. Die Herstellung erfolgt in der Weise, daß zunächst eine wässrige Mischung der genannten Bestandteile zubereitet wird, wobei als Multipolymer beispielsweise wässrige Emulsionen von Ethylenvinylacetat-Copolymeren verwendet werden.

EP-A-279982 betrifft transdermale Formulierungen zur Empfängnisverhütung. Diese Formulierungen weisen eine Polymermatrix auf, welche die Wirkstoffe sowie relativ hohe Anteile eines bestimmten Hautpermeations-Enhancers (Glycerinmonooleat) enthält. Die Polymermatrix wird bevorzugt aus Ethylenvinylacetat-Copolymeren hergestellt; sie kann Zusatzstoffe wie z. B. Vaseline enthalten. Hinsichtlich der Herstellung von transdermalen therapeutischen Systemen wird lediglich in allgemeiner Weise und ohne nähere Angaben darauf hingewiesen, daß diese unter Verwendung von Lösungen oder Schmelzen erfolgen kann.

EP-A-0305756 ist ausschließlich auf Haftschmelzkleber und deren Verwendung in Vorrichtungen zur Abgabe von Stoffen gerichtet. Typische Zusammensetzungen dieser Haftschmelzkleber enthalten 10-100 Gew.-% Polymer, 20-80 Gew.-% Weichmacher und 10-80 Gew.-% Klebrigmacher. Als Polymer kann beispielsweise Polyethylen-Vinylacetat verwendet werden.

GB-A-2037659 beschreibt eine biegsame Faserplatte, die mit einer thermoplastischen Zusammensetzung imprägniert ist, welche bei 40-220 °C schmilzt. Die thermoplastische Zusammensetzung enthält eine homogene Mischung aus mindestens einem synthetischen, linearen thermoplastischen Polymer und mindestens einem Wachs und/oder klebrigmachenden Harz. Als thermoplastische Polymere kommen unter anderem Ethylenvinylacetat-Copolymere in Betracht.

EP-A-0379045 offenbart eine haftklebende transdermale Zusammensetzung, welche einen Wirkstoff, ein Multipolymer, einen Kautschuk und einen Klebrigmacher enthält. Das Multipolymer umfaßt ein Ethylenvinylacetat-Polymer und ein Acrylat-Polymer. Um die Löslichkeit des Wirkstoffs in dem Polymer zu verbessern, können Cosolventien wie z. B. Lecithin, Retinolderivate, Tocopherol, Fettsäuren, Mineralöl, etc. hinzugefügt werden. Die Herstellung erfolgt in der Weise, daß zunächst eine homogene Mischung der genannten Bestandteile erzeugt wird, wobei das Ethylenvinylacetat-Polymer in Form von wässrigen Emulsionen hinzugefügt wird.

Bei anderen TTS-Typen werden Copolymere von Ethylen und Vinylacetat (EVA-Copolymere, z. B. das Handelsprodukt EVATANE^{®}) als Grundmaterial für das Wirkstoffreservoir eingesetzt, z. B. im TTS "Nicoderm CQ" oder im TTS "Testoderm 15" für die Wirkstoffe Nikotin bzw. Testosteron. Da EVA-Copolymere jedoch über keine Autoadhäsion verfügen, d. h. nicht haftklebend sind, muß die Hauthaftung bei solchen TTS durch eine zusätzliche haftklebende Schicht gewährleistet werden. EVA-Copolymere werden in bestimmten TTS ferner auch in Form von Membranen eingesetzt, welche als Steuermembran zur Kontrolle der Wirkstofffreisetzung dienen ("membrankontrolliertes TTS"). Bei diesem TTS-Typ erfolgt die Steuerung der Arzneistoffabgabe durch eine arzneistoffspezifische Porengröße (mikroporöse Steuermembran) oder durch die Membrandicke oder Membranzusammensetzung, z. B. EVA-Steuermembran. Allerdings weisen auch EVA-Steuermembranen, wie Wirkstoff-Reservoire auf EVA-Basis, keine haftklebenden Eigenschaften auf.

Der Erfindung lag deshalb die Aufgabe zugrunde, Haftkleberzusammensetzungen bereitzustellen, die gute haftklebende Eigenschaften aufweisen, chemisch möglichst inert sind, insbesondere in Bezug auf Arzneimittelwirkstoffe, und die die vorstehend genannten vorteilhaften Eigenschaften der bekannten Polyacrylat-Haftkleber aufweisen. Zugleich wird gefordert, daß kein Zusatz von Vernetzungsreagenzien zwecks Erhöhung der inneren Kohäsion erforderlich ist. Ferner soll gleichzeitig der Anteil von polaren oder funktionellen Gruppen im Polymergarüst minimiert werden. Die Haftkleberzusammensetzungen sollen außerdem eine Steuerung der Wirkstofffreisetzung ermöglichen.

Überraschenderweise wird diese Aufgabe durch Haftklebermassch auf der Basis von Ethylen-Vinylacetat(EVA)-Copolymeren gelöst, welche gemäß Anspruch 1 einen Zusatz von Klebharz(en) aufweisen.

Folglich enthält eine erfindungsgemäße Haftklebermasse eine Polymerkomponente (A), bei der es sich um ein Ethylen-Vinylacetat-Copolymer oder eine Kombination von mindestens zwei Ethylen-Vinylacetat-Copolymeren handelt, und eine Komponente (B), bei der es sich um ein Klebharz oder eine Kombination von Klebharzen handelt; der Anteil der Komponente B beträgt dabei bis zu 55 Gew.-%, bezogen auf die Summe der Komponenten (A) und (B), ohne Wirk- oder sonstige Hilfsstoffe (d. h. bezogen auf den reinen Haftkleber).

Die Komponente (A) and (B) sind in geläster From in einen Lösemittelgemisch gemäß Anspruch 1 enthalten.

Die vorliegende Erfindung beruht auf der überraschenden Beobachtung, daß man neuartige Haftklebermassen erhält, wenn man EVA-Copolymere durch die in Anspruch 1 genannten organischen Lösungsmittel gemische unter Erwärmung in Lösung bringt und sodann Klebharzen in einem bestimmten Mischungsverhältnis hinzufügt; ein Zusatz von Vernetzern oder Weichmachern ist dabei nicht erforderlich.

von Vorteil ist dabei, daß der Klebharz-Anteil relativ niedrig ist, im Vergleich zu Haftklebern auf der Basis von synthetischen Kautschukpolymeren (z. B. Styrol-Isopren-Styrol(SIS)-Blockcopolymere oder Styrol-Butadien-Styrol(SBS)-Blockcopolymere), welche deutlich über 60 Gew. -% an Klebharzzusätzen benötigen, um ausreichende haftklebende Eigenschaften zu erhalten. Ein derart hoher Klebharz-Anteil kann jedoch zu Hautreizungen führen.

Hingegen wird bei den erfindungsgemäßen Haftklebern ein Klebharzanteil von höchstens 55 Gew.-% (bezogen auf die Summe der Komponenten A und B, ohne Wirk- oder sonstige Hilfsstoffe) benötigt, um die geforderten Haftkleber-Eigenschaften zu erreichen. Auf diese Weise kann das Hautreizungspotential niedrig gehalten werden. Besonders bevorzugt ist ein Klebharz-Anteil im Bereich von 45 bis 55 Gew.-%; Klebstoffe mit dieser Zusammensetzung weisen besonders gute Haftkleb-Eigenschaften auf. Der Klebharz-Anteil (Komponente B) sollte jedoch mindestens 25 Gew.-%, besser noch mindestens 30 Gew.-% betragen.

Klebharz-Zusätze sind dem Fachmann als hydrierte Harzsäurederivate bekannt. Hydrierte Harzsäuren oder deren Derivate werden seit längerer Zeit als Grundmaterialien in Heftpflastern verwendet. Harzsäuren sind im Naturprodukt Kolophonium enthalten, das durch Destillation des Nadelholzbalsams oder bei der Extraktion von Nadelholzstubben vorwiegend subtropisch-mediterraner Klimazonen gewonnen wird. Für die Herstellung der erfindungsgemäßen Haftkleber werden bevorzugt Harzsäuren verwendet, die zum Schutz gegen Sauerstoffeinflüsse und zur Erhöhung der chemischen Inertheit partiell oder voll hydriert wurden und die zwecks Verbesserung der Alkalistabilität und ebenfalls zur Erhöhung der chemischen Inertheit an ihrer Carboxylgruppe verestert wurden. Besonders bevorzugt sind dabei Methylester, Glycerinester, Pentaerythritester, maleinsäure-modifizierte Pentaerithritester, maleinsäure-modifizierte Glycerinester oder Triethylenglycolester hydrierter Harzsäuren. Daneben können auch andere hautverträgliche Derivate des hydrierten Kolophoniums verwendet werden, sowie entsprechende Ester der nicht hydrierten Harzsäuren oder des nicht hydrierten Kolophoniums.

Als Komponente A kommen bevorzugt solche Ethylen-Vinylacetat-Copolymere in Betracht, die einen Vinylacetat-Gehalt von mindestens 28 Gew.-% aufweisen, bezogen auf die Monomer-Zusammensetzung. Der Einbau des im Vergleich zu Ethylen stärker polaren Vinylacetat-Monomers führt zu einer Reduzierung der Glasübergangstemperatur und damit verbunden zu einer Verringerung kristalliner Anteile in den EVA-Copolymeren. Dadurch sinkt die Viskosität, die Quellbarkeit steigt, und die genannten EVA-Copolymere können mit den oben beschriebenen Klebharzen in geeigneten Lösemittelgemischen, oder auch aus der Schmelze, adhäsive und gut verarbeitbare (d. h. flüssige oder verstreichbare) Haftklebermassen bilden.

Die besten Ergebnisse werden erzielt, wenn der Anteil des/der EVA-Copolymere im Bereich von 46 bis 55 Gew.-% liegt, bezogen auf die Summe der Komponenten (A) und (B). Vorzugsweise beträgt der Anteil der Komponente (A) mindestens 25 Gew.-%, insbesondere mindestens 15 Gew.-%.

Die erfindungsgemäßen Haftklebermassen werden, wie beschrieben, auf der Basis von EVA-Copolymeren und Klebharzen hergestellt, welche die Grundbestandteile dieser Haftkleber darstellen. Zur Herstellung von Haftkleberschichten oder wirkstoffhaltigen Schichten können Wirkstoffe sowie Zusatzstoffe wie z. B. hautpenetrationsfördernde Stoffe zugesetzt werden. Vorzugsweise beträgt der Haftkleber-Anteil in einer wirkstoffhaltigen Schicht oder Wirkstoffmatrix mindestens 60 Gew.-%, besonders bevorzugt mindestens 75 Gew.-%.

Dagegen ist bei den erfindungsgemäßen Haftklebern ein Zusatz von Weichmachern oder Vernetzern zur Einstellung der Adhäsions- und Kohäsionsbalance nicht erforderlich; deshalb wird gemäß einer bevorzugten Ausführungsform auf derartige Zusätze verzichtet. Vorzugsweise werden den erfindungsgemäßen Haftklebern auch keine andersartigen haftklebenden Polymere, wie z. B. Polyacrylate, zugesetzt.

Die erfindungsgemäßen Haftkleber lassen sich in vielfältiger Weise zur Herstellung von wirkstofffreien oder wirkstoffhaltigen, auf der Haut haftklebenden Schichten verwenden. Insbesondere eignen sie sich zur Herstellung von wirkstoffhaltigen Haftkleberschichten oder Klebstoffmatrices zur Wirkstofffreisetzung im Bereich der Human- oder Veterinärmedizin, z. B. als Bestandteil von transdermalen therapeutischen Systemen. Daneben können diese Haftkleber auch bei der Herstellung von Wundschnellverbänden, Fixierpflastern oder selbstklebenden Elektroden verwendet werden, oder auch zur Herstellung haftklebender Schichten von solchen TTS, die an sich nicht adhäsiv sind.

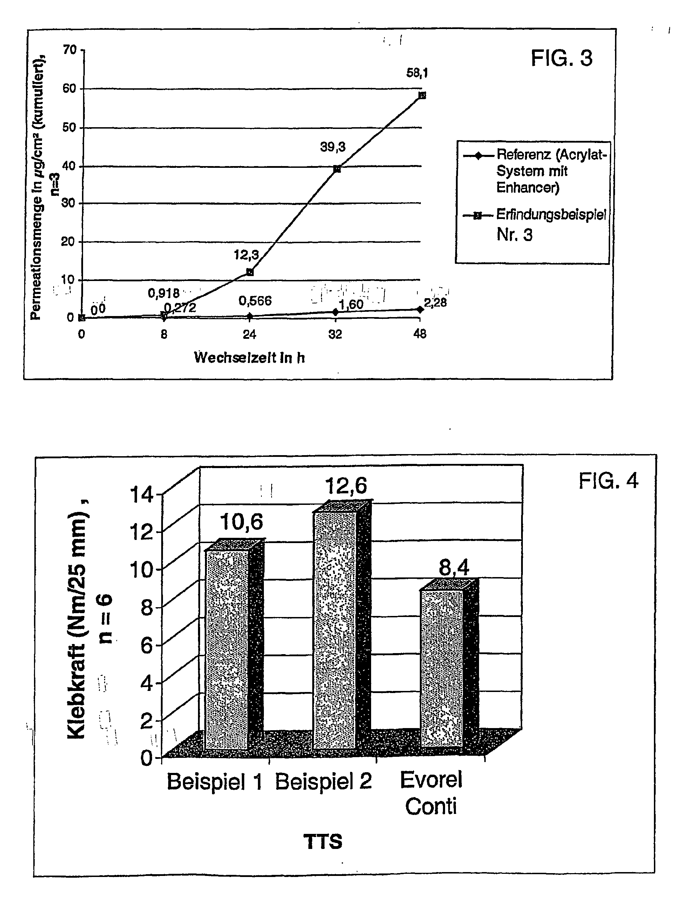

Aufgrund ihrer vorteilhaften Eigenschaften und ihrer ausreichenden Adhäsion (FIG. 4) sind die erfindungsgemäßen Haftkleber besonders für die Herstellung der Wirkstoffmatrix transdermaler therapeutischer Systeme geeignet. Vorzugsweise wird ein solches System als einschichtiges Matrix-System gestaltet, das aus einer im wesentlichen wirkstoffundurchlässigen Rückschicht, der eigentlichen aktiven, wirkstoffhaltigen Matrixschicht, sowie einer wiederablösbaren Schutzschicht besteht. Ein derartig konzipiertes TTS-Matrixsystem zeichnet sich durch eine relativ einfache und kostengünstige Herstellung aus. Ferner wird bevorzugt, daß die Wirkstoffmatrix ein einphasiges System darstellt.

Vorzugsweise weisen die erfindungsgemäßen TTS einen Wirkstoffanteil im Bereich von 0,1 bis 50 Gew.-% auf, bezogen auf die Haftkleberschicht oder Wirkstoffmatrix. Gemäß einer weiteren bevorzugten Ausführungsform beträgt der Klebharzanteil in den TTS weniger als 50 Gew.-%, besonders bevorzugt 39 bis 49 Gew.-%, bezogen auf die Haftkleberschicht oder Wirkstoffmatrix.

Durch die erfindungsgemäße Kombination von EVA-Copolymeren mit Klebharz(en) zu einer homogenen, einphasigen Haftklebermatrix können die Vorteile von Matrix-kontrollierten und membrankontrollierten Reservoirsystemen in einem System vereinigt werden. Dies stellt gegenüber dem Stand der Technik einen wesentlichen Vorteil dar, weil die Steuermembran (aufgrund fehlender Klebfähigkeit) in membrankontrollierten Systemen gewöhnlich mit einer zusätzlichen haftklebenden Schicht oder aber mit einem haftklebenden Rand (Überpflaster) ausgestattet sein muß, wodurch die Herstellung aufwendiger wird.

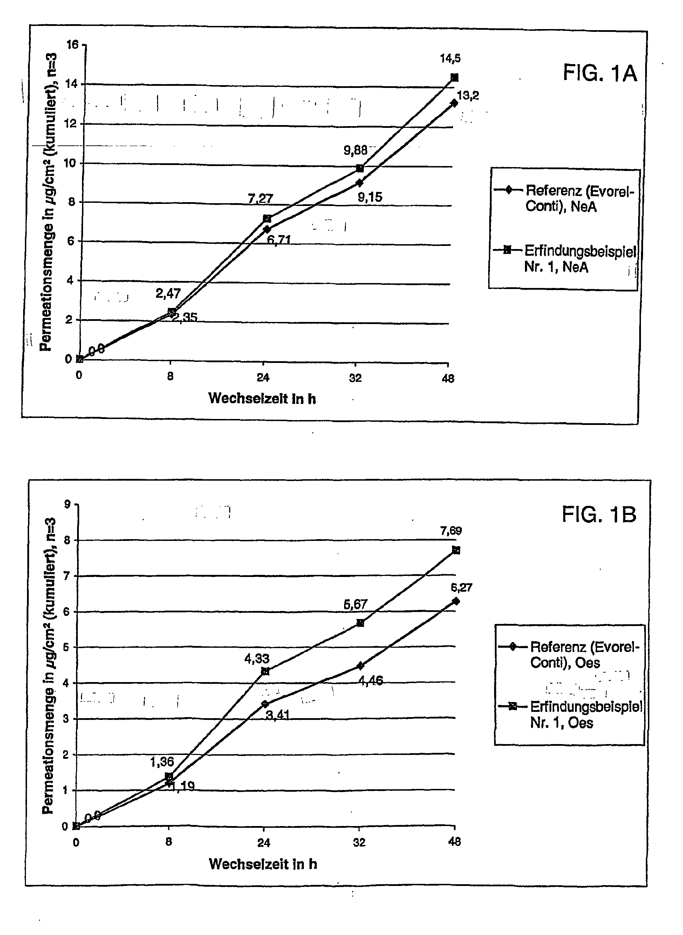

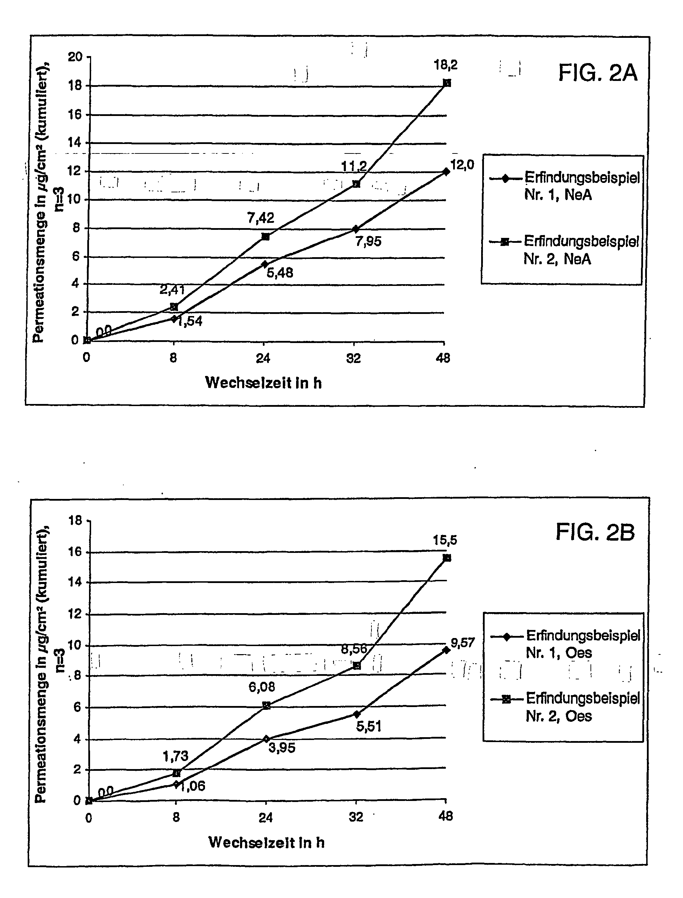

In diesem Zusammenhang ist von besonderem Vorteil, daß durch Auswahl unterschiedlicher Anteile von Vinylacetat bzw. Ethylen im EVA-Copolymer (Komponente A) die Wirkstoffdiffusion bzw. Wirkstoff-Abgabe gesteuert werden kann (vgl. Fig. 1 A,B und Fig. 2 A,B).
Aufgrund dieser Eigenschaft der erfindungsgemäßen Haftkleber können diese auch verwendet werden, um Steuermembranen herzustellen, welche im Gegensatz zu bekannten Steuermembranen haftklebend sind. Derartige haftklebende EVA-Steuermembranen oder Steuerschichten können als Steuermembran in membrankontrollierten Reservoirsystemen oder als zusätzlicher Hautstrich (Hautkontaktschicht) in matrixkontrollierten TTS verwendet werden, sofern die Matrix selbst über keine adhäsiven Eigenschaften verfügt.

Die erfindungsgemäßen Haftkleber können folglich zur Herstellung von wirkstoffhaltigen oder wirkstofffreien Schichten von TTS, wie auch von Steuermembranen oder -Schichten, verwendet werden. Die Erfindung betrifft deshalb TTS mit einem an sich bekannten Aufbau, umfassend eine wirkstoffhaltige Matrix oder ein Wirkstoffreservoir, eine Rückschicht und eine ablösbare Schutzschicht, wobei diese TTS mindestens eine wirkstoffhaltige oder wirkstofffreie Schicht enthalten, die aus einem erfindungsgemäßen Haftkleber hergestellt ist oder einen solchen enthält.

Der Aufbau der erfindungsgemäßen TTS umfaßt neben einer Wirkstoffmatrix eine wirkstoffundurchlässige Rückschicht sowie eine ebenfalls wirkstoffundurchlässige, abziehbare Schutzfolie.
Als Rückschicht eignen sich vor allem Polyester, welche sich durch besondere Festigkeit auszeichnen, darüber hinaus aber auch andere hautverträgliche Kunststoffe, wie z. B. Polyvinylchlorid, Ethylenvinylacetat, Vinylacetat, Polyethylen, Polypropylen, Cellulosederivate und viele andere mehr. Im Einzelfall kann die Rückschicht mit einer zusätzlichen Auflage versehen werden, z. B. durch Bedampfung mit Metallen oder anderen diffusionssperrenden Zusatzstoffen wie Siliciumdioxid, Aluminiumoxid oder ähnlicher Stoffe, die dem Fachmann bekannt sind.
Für die ablösbare Schutzfolie können dieselben Materialien verwendet werden wie für die Rückschicht, vorausgesetzt, daß sie durch geeignete Oberflächenbehandlung, wie z. B. Silikonisierung, ablösbar ist. Es können aber auch andere ablösbare Schutzschichten, wie Polytetrafluorethylen-behandeltes Papier, Cellophan, Polyvinylchlorid, oder ähnliche verwendet werden.

Neben dem/den aktiven Wirkstoff (en) kann die erfindungsgemäße haftklebende Matrix optional penetrationsfördernde Stoffe enthalten, die dem Fachmann grundsätzlich bekannt sind. Zu der erfindungsgemäßen Haftklebermatrix kompatible und deshalb bevorzugte Penetrationsförderer sind beispielsweise gesättigte oder ungesättigte Fettsäuren, geradkettige oder verzweigte Fettalkohole sowie deren Ester, mehrwertige aliphatische Alkohole, Polyethylenglykole, Sorbitanfettsäureester sowie deren durch Ethoxylierung erhältlichen Derivate oder Fettalkohol-Ethoxylate, oder monocyclische Monoterpene. Der Zusatz von penetrationsfördernden Stoffen erfolgt bevorzugt in einer Konzentration von 0,1 bis 30 Gew.-%, bezogen auf die Haftkleberschicht oder Wirkstoffmatrix.

Es hat sich herausgestellt, daß die erfindungsgemäßen Haftkleberschichten auf der Basis von EVA-Copolymeren und Klebharzen besonders für die transdermale Abgabe von Hormonen, insbesondere von Steroidhormonen wie Sexualsteroiden geeignet sind, insbesondere für natürliche und/oder synthetische Estrogene und Gestagene, sowohl einzeln als auch in Kombination, z. B. Estrogen/Gestagen-Kombinationen. Besonders vorteilhaft ist dabei, daß die erfindungsgemäßen Systeme keinerlei Neigung zu einer Rekristallisation des enthaltenen Wirkstoffs zeigen. Die Wirkstoffmatrix kann ein oder mehrere Estrogene, oder Estrogen(e) in Kombination mit mindestens einem Gestagen, enthalten. Die gesamte Konzentration dieser Hormone beträgt hierbei 0,1 bis 15 Gew.-%, bezogen auf die Wirkstoffmatrix. Im Falle einer Estrogen-Gestagen-Kombination hat es sich als besonders vorteilhaft erwiesen, wenn Estrogen(e) und Gestagen(e) in einem molaren Verhältnis von 1:1 bis 1:10 vorliegen.

Aufgrund der erwähnten Eigenschaften der erfindungsgemäßen Haftkleber ist dieser nicht nur als Matrix für neutrale Arzneimittel-Wirkstoffe (Fig. 1A,B und Fig. 2A,B) geeignet, sondern insbesondere auch für die Herstellung von wirkstoffhaltigen Schichten, welche hydrolyse-anfällige(n) Wirkstoff(e) enthalten. Hierzu gehören insbesondere Wirkstoffmoleküle mit Acetylfunktion, vorzugsweise Acetylsalicylsäure oder Diamorphin, und saure oder basische, oder organische, ionische (Fig. 3) Arzneimittelwirkstoffe oder deren pharmazeutisch akzeptablen Salze.

Wegen der chemischen Inertheit der erfindungsgemäßen Haftkleber (aufgrund fehlender polarer oder funktioneller Gruppen) gegenüber Arzneimittelwirkstoffen werden chemische Wechselwirkungen mit den Wirkstoffen, wie irreversible Säure-Base-Reaktionen, weitestgehend ausgeschlossen. Folglich kann die Wirkstofffreisetzung und damit die Bioverfügbarkeit nicht durch derartige unerwünschte Reaktionen beeinträchtigt werden, anders als bei Haftklebermatrices auf der Basis von Acrylatklebern.
Ferner weisen die erfindungsgemäßen Haftkleber ein gutes Reservoirvermögen auf, weil sich die meisten Wirkstoffe, sowohl lipophile als auch hydrophile, in EVA-Copolymeren gut lösen. Beispielsweise sind Wirkstoff-Beladungen von 10 Gew.-% (bezogen auf die Wirkstoffmatrix) ohne Zuhilfenahme von Lösungsvermittlern erreichbar, z. B. mit dem stark hydrophilen organischen Morphinium-Salz Morphinium-N-Acetylglycinat (Beispiel 3) oder mit dem stark lipophilen Gestagen Norethisteronacetat. Derart hohe Beladungskapazitäten sind mit Haftklebern auf der Basis von Polyisobutylen oder mit Silikonklebern nicht erreichbar (Beladungskapazität maximal 5 Gew.-%); letztere sind zudem noch sehr teuer.

Die Herstellung der erfindungsgemäßen Haftkleber und Haft der in Anspruch 1 genannten kleberschichten erfolgt aus der Lösung unter Verwendung organishen Lösungsmittel gemische.

Zur Herstellung der erfindungsgemäßen Haftklebermassen aus einer Lösung wird/werden Ethylen-Vinylacetat-Copolymer(e) (Komponente A) bei erhöhter Temperatur (vorzugsweise 40-75 °C, insbesondere 45-55 °C) in einem organischen Lösungsmittelgemisch unter Rühren gelöst. Dabei wird ein Benzin- und Propylacetat enthaltendes Lösemittelgemisch verwendet oder ein Gemisch aus der folgenden Gruppe: Benzin/Propylacetat (2:1), Benzin/Butanon (1:1), Benzin/Propylacetat/Butanon (1:1:1), Hexan/Propylacetat/Aceton (1:1:1) (jeweils Volumenanteile). Anschließend wird/werden Klebharz(e) (Komponente B) unter Rühren und Homogenisierung bis zum vollständigen Auflösen eingearbeitet, ebenfalls bei erhöhter Temperatur wie angegeben. Die Zugabe von optionalen Bestandteilen (z. B. Wirkstoffe, Enhancer) und das Beschichten erfolgt wie oben beschrieben. Zum Entfernen der Lösemittel wird die so erhaltene Schicht einer Trocknung bei erhöhter Temperatur unterworfen (bevorzugt bei ca. 30-80 °C, insbesondere bei ca. 50 °C).

Im folgenden wird die Erfindung anhand von Beispielen und Abbildungen weiter erläutert.
Mit den hergestellten erfindungsgemäßen wirkstoffhaltigen Haftmatrix-Systemen wurden Permeationsmessungen am invitro-Hautmodell unter Verwendung von Humanhaut-Epidermis (Fig. 1 - 3) bzw. mit Hilfe modifizierter Franz-Diffusionszellen durchgeführt. Als Akzeptormedium diente in allen Fällen isotonische Kochsalzlösung mit 0.1 % Zusatz von NaN3 als Konservierungsmittel, thermostatisiert auf 37 °C.

### Beispiel 1:

### Herstellung eines selbstklebenden, wirkstoffhaltigen Films (17-β-Estradiol und Norethisteronacetat)

In 80 g eines Lösemittelgemisches, bestehend aus 2 Teilen Spezialbenzin Typ 80/110 und 1 Teil Propylacetat, werden 54 g eines EVA-Copolymeren mit 40 Gew.-% Vinylacetat und einem Schmelzindex von 55 (EVATANE 40/55^{®}) eingetragen und unter Rühren sowie Wärmezufuhr bei 50 °C gelöst. Nach ca. 30 min Rührzeit wurde eine viskose, farblos bis leicht trübe Lösung erhalten. In diese Lösung wurden anschließend 66 g des Klebharzes Foral^{®} 85 E eingetragen und bis zum vollständigen Auflösen (ca. 15 min) bei ebenfalls 50 °C gerührt. Es resultierte eine 50%ige, niedrig viskose, gelbliche und leicht trübe Lösung (Kleberlösung I), die auch nach dem Erkalten noch als rührfähige Kleberlösung vorlag.
Für die Herstellung des selbstklebenden, wirkstoffhaltigen Films wurden 9,88 g Kleberlösung I vorgelegt, in die unter Rühren portionsweise 2,4 g 17-β-Estradiol und anschließend 9,6 g Norethisteronacetat eingetragen wurden. Falls erforderlich, können zur Erniedrigung der Viskosität des Masse-Ansatzes 1000 µl Methanol zugegeben werden. Der Masse-Ansatz wird insgesamt 30 min bei einer Rührgeschwindigkeit von 350 U/min homogenisiert. Anschließend erfolgt eine viertelstündige Entgasung bei 45 °C im Ultraschallbad, um überschüssige Luft aus der Masse zu entfernen.
Die wirkstoffhaltige Kleberlösung wird dann mit Hilfe einer Ausziehrakel in einer Naßschichtdicke von 300 µm auf einer silikonisierten Polyethylenterephthalat-Folie ausgestrichen. Danach werden die Lösemittel durch halbstündiges Trocknen bei 50 °C in einem Trockenschrank mit Abluftführung entfernt. Der lösemittelfreie, wirkstoffhaltige Kleberfilm wird abschließend mit einer 15 µm dicken Polyesterfolie (als Rückschicht) durch Aufkaschieren abgedeckt. Der Kleberanteil in der Matrix nach Fertigungsende beträgt 88 Gew.-%; davon entfallen 48,4 Gew.-% auf den Klebharzanteil.

### Beispiel 2:

### Herstellung eines selbstklebenden, wirkstoffhaltigen Films (17-β-Estradiol und Norethisteronacetat)

In 60 g eines Lösemittelgemisches, bestehend aus 2 Teilen Spezialbenzin Typ 80/110 und 1 Teil Propylacetat, werden 69 g eines EVA-Copolymeren mit 33 Gew.-% Vinylacetat und einem Schmelzindex von 400 (EVATANE 33/400^{®}) eingetragen und unter Rühren sowie Wärmezufuhr bei 50 °C gelöst. Nach ca. 30 min Rührzeit wurde eine viskose, farblos bis leicht trübe Lösung erhalten. In diese Lösung wurden anschließend 56 g des Klebharzes Foral^{®} 85 E eingetragen und bis zum vollständigen Auflösen (ca. 15 min) bei ebenfalls 50 °C gerührt. Es resultierte eine 58%ige, niedrig viskose, gelbliche und leicht trübe Lösung (Kleberlösung II), die auch nach dem Erkalten noch als rührfähige Kleberlösung vorlag.

Für die Herstellung des selbstklebenden, wirkstoffhaltigen Films wurden 7,59 g Kleberlösung II vorgelegt, in die unter Rühren portionsweise 2,4 g 17-β-Estradiol und anschließend 9,6 g Norethisteronacetat eingetragen wurden.
Die weitere Herstellung des Kleberfilms erfolgte wie unter Beispiel 1 beschrieben. Der Kleberanteil in der Matrix nach Fertigungsende beträgt 88 Gew.-%; davon entfallen 39,6 Gew.-% auf den Klebharzanteil.

### Beispiel 3:

### Herstellung eines selbstklebenden, wirkstoffhaltigen Films (Morphinium-N-Acetylglycinat als Wirkstoff)

Die Herstellung erfolgte wie unter Beispiel 1 beschrieben, jedoch mit einem Klebharz-Anteil von 49,5 Gew.-% in der Matrix und Morphinium-N-Acetylglycinat (10 Gew.-%) als Wirkstoff. Als EVA-Copolymer wurde wiederum EVATANE 40/55^{®} verwendet.

**Tabelle 1:**

| Wirkstoffhaltige Filme nach den Beispielen 1 bis 3 | | | |
|---|---|---|---|
| | Beispiel 1 | Beispiel 2 | Beispiel 3 |
| EVA-Copolymer-Typ | 40/55 | 33/400 | 40/55 |
| Vinylacetat-Gehalt im Copolymer (Gew.-%) | 40 | 33 | 40 |
| EVA-Anteil in der Matrix (in Gew.-%) | 39,6 | 48,4 | 40,5 |
| Klebharz-Anteil in der Matrix (Gew.-%) | 48,4 | 39,6 | 49,5 |
| Vinylacetat-Anteil in der Matrix (Gew.-%) | 15,84 | 15,972 | 16,20 |
| Ethylen-Anteil in der Matrix (Gew.-%) | 23,76 | 32,428 | 24,3 |
| Wirkstoff(e) (Gew.-%) | Oes: 2,4 NeA: 9,6 | Oes: 2,4 NeA: 9,6 | Morphinium-N-Acetylglycinat: 10 |

### Erläuterung der Abbildungen

Fig. 1 bis 3 geben die Ergebnisse der Permeationsmessungen wieder, die mit den Beispielen 1 bis 3 durchgeführt wurden.
Fig. 1A
   zeigt Ergebnisse von Permeationsmessungen mit einer erfindungsgemäßen rekristallisationsfreien Matrix nach Beispiel 1 mit 48,4 Gew.-% Klebharzanteil und 17-β-Estradiol (2,4 Gew.-%; als Semihydrat) und Norethisteronacetat (9,6 Gew.-%) als Wirkstoffe, ohne Zusatz von Enhancer, Vernetzer, Weichmacher, Kristallisationsinhibitoren oder sonstigen Hilfsstoffen.
   Als Vergleichsbeispiel diente ein handelsübliches Referenz-Kombi-TTS (Evorel Conti^{®}; Cilag, CH), das 2,5 Gew.-% 17-β-Estradiol (als Semihydrat), 8,75 Gew.-% Norethisteronacetat, 2 Gew.-% Myprogat 90 und 86,75 Gew.-% Duro-Tak 2287 enthält. Myprogat 90 ist ein wasserabsorbierendes Mittel, kein Enhancer.
   Fig. 1A zeigt einen Vergleich der kumulierten Permeationswerte von Norethisteronacetat (Abkürzung: "NeA").
Fig. 1B:
   zeigt Ergebnisse von Permeationsmessungen mit einer erfindungsgemäßen Matrix nach Beispiel 1, wie in Fig. 1A, jedoch wird hier ein Vergleich der kumulierten Permeationswerte von 17-β-Estradiol (Abkürzung: "Oes") dargestellt.
Fig. 2A:
   zeigt Ergebnisse von Permeationsmessungen mit einer erfindungsgemäßen Matrix nach Beispiel 2 mit 39,6 Gew.-% Klebharzanteil, 15,972 Gew.-% Vinylacetatanteil (bezogen auf die gesamte Matrix), sowie 17-β-Estradiol (2,4 Gew.-%; als Semihydrat) und Norethisteronacetat (9,6 Gew.-%) als Wirkstoffe, ohne Zusatz von Enhancer, Vernetzer, Weichmacher, Kristallisationsinhibitoren oder sonstigen Hilfsstoffen. Als Vergleichsbeispiel diente eine erfindungsgemäße Matrix nach Beispiel 1 mit annähernd gleichem Vinylacetat-Anteil (15,84 Gew.-%), aber geringerem Ethylengehalt (23,76) Gew.-%) und höherem Klebharz-Anteil (48,4 Gew.-%).
   Fig. 2A zeigt einen Vergleich der kumulierten Permeationswerte von Norethisteronacetat ("NeA").
Fig. 2B:
   zeigt Ergebnisse von Permeationsmessungen mit einer erfindungsgemäßen Matrix nach Beispiel 2, wie in Fig. 2A, jedoch wird hier ein Vergleich der kumulierten Permeationswerte von 17-β-Estradiol ("Oes") dargestellt.
Fig. 3:
   zeigt Ergebnisse von Permeationsmessungen mit einer erfindungsgemäßen Matrix nach Beispiel 3 mit 49,5 Gew.-% Klebharzanteil und Morphinium-N-Acetylglycinat als Wirkstoff, ohne Zusatz von Enhancer, Vernetzer, Weichmacher oder sonstigen Hilfsstoffen.
   Als Vergleichsbeispiel (Referenz) diente eine Haftklebermatrix auf Basis von Polyacrylaten mit Vernetzer und mit 25 Gew.-% Ölsäure als Enhancer. Die Wirkstoffbeladung war in beiden Fällen gleich hoch (10 Gew.-%).
Fig. 4
   zeigt einen Vergleich der Klebkraftwerte, die an erfindungsgemäßen TTS auf Basis von EVA-Copolymeren und Klebharzen nach Beispiel 1 und 2 gemessen wurden, im Vergleich mit den Klebkraftwerten eines Referenz-TTS (Evorel Conti^{®}) auf Acrylatbasis.
   Die Messungen beruhen auf dem Prüfverfahren nach "Peel Adhesion 90° Test" (Prüfplatte Aluminium; Abzugsgeschwindigkeit 300 mm/min; Zugprüfmaschine nach DIN 51221, Teil 1).

## Patentansprüche

1. Haftklebermasse zur Herstellung von Haftkleberschichten für medizinische Anwendungszwecke auf der Basis von Ethylen-Vinylacetat-Copolymeren, **dadurch gekennzeichnet, daß** die Haftklebermasse
- als Polymerkomponente (A) ein Ethylen-Vinylacetat-Copolymer oder eine Kombination von mindestens zwei Ethylen-Vinylacetat-Copolymeren,
- als Komponente (B) ein Klebharz oder eine Kombination von Klebharzen in einem Anteil von bis zu 55 Gew.-%, bezogen auf die Summe der Komponenten (A) und (B) ohne Wirk- oder sonstige Hilfsstoffe, enthält und die Komponenten (A) and (B) in gelösten Form in einem Benzin und Propylacetat enthaltenden organischen Lösemittelgemisch oder in einen aus der Gruppe Benzin/Propylacetat (2:1), Benzin/Butanon (1:1), Benzin/Propylacetat/Butanon (1:1:1), Hexan/Propylacetat/ Aceton (1:1:1); ausgewählten Lösemittelgemisch enthalten sind, wobei die angegebenen Mischungsverhältnisse sich auf Volumenanteile beziehen.

2. Haftklebermasse nach Anspruch 1, **dadurch gekennzeichnet, daß** das/die Ethylen-Vinylacetat-Copolymer(e) (Komponente A) einen Vinylacetat-Gehalt von mindestens 28 Gew.-% aufweist/aufweisen, bezogen auf die Monomer-Zusammensetzung.

3. Haftklebermasse nach Anspruch 1 oder 2, **dadurch gekennzeichnet, daß** die Komponente (B) in einem Anteil von mindestens 25 Gew.-%, vorzugsweise von mindestens 30 Gew.-%, am meisten bevorzugt in einem Anteil von 45 bis 55 Gew.-% enthalten ist, bezogen auf die Summe der Komponenten (A) und (B).

4. Haftklebermasse nach einem der Ansprüche 1 bis 3, **dadurch gekennzeichnet, daß** die Komponente (A) in einem Anteil von mindestens 15 Gew.-%, vorzugsweise von mindestens 25 Gew.-%, am meisten bevorzugt in einem Anteil von 46 bis 55 Gew.-% enthalten ist, bezogen auf die Summe der Komponenten (A) und (B).

5. Haftklebermasse nach Anspruch 1 oder 2, **dadurch gekennzeichnet, daß** die Komponente (A) in einem Anteil von 46 bis 55 Gew.-% und die Komponente (B) in einem Anteil von 45 bis 55 Gew.-% enthalten ist, jeweils bezogen auf die Summe der Komponenten (A) und (B).

6. Haftklebermasse nach einem der Ansprüche 1 bis 5, **dadurch gekennzeichnet, daß** das/die Klebharz(e) aus der Gruppe ausgewählt ist/sind, welche Ester des Kolophoniums und Ester des hydrierten Kolophoniums umfaßt, besonders bevorzugt aus der aus Methylestern, Glycerinestern, Pentaerythritestern, maleinsäure-modifizierten Pentaerythritestern, maleinsäure-modifizierten Glycerinestern und Triethylenglycolestern bestehenden Gruppe.

7. Haftklebermasse nach einem der Ansprüche 1 bis 6, **dadurch gekennzeichnet, daß** sie keine(n) Weichmacher und/oder Vernetzer enthält.

8. Wirkstoffhaltige Haftkleberschicht oder Klebstoffmatrix zur Wirkstofffreisetzung für die Human- oder Veterinärmedizin, **dadurch gekennzeichnet, daß** sie aus einer Haftklebermasse nach einem der Ansprüche 1 bis 7 hergestellt ist.

9. Transdermales therapeutisches System mit einer wirkstoffhaltigen Matrix, einer Rückschicht und einer ablösbaren Schutzschicht, **dadurch gekennzeichnet, daß** es mindestens eine wirkstoffhaltige oder wirkstofffreie Schicht aufweist, die aus einer Haftklebermasse nach einem der Ansprüche 1 bis 7 hergestellt ist.

10. Transdermales therapeutisches System nach Anspruch 9, **dadurch gekennzeichnet, daß** die Wirkstoffmatrix einphasig und vorzugsweise einschichtig ist.

11. Transdermales therapeutisches System nach Anspruch 9 oder 10, **dadurch gekennzeichnet, daß** die Wirkstoffabgabe durch den Vinylacetat-Gehalt des/der Ethylen-Vinylacetat-Copolymers/Copolymeren der Wirkstoffmatrix gesteuert wird.

12. Haftkleberschicht, Wirkstoffmatrix oder transdermales therapeutisches System nach einem der Ansprüche 8 bis 11, **dadurch gekennzeichnet, daß** der Wirkstoffanteil, bezogen auf die Haftkleberschicht oder Wirkstoffmatrix, 0,1 bis 50 Gew.-% beträgt.

13. Haftkleberschicht, Wirkstoffmatrix oder transdermales therapeutisches System nach einem der Ansprüche 8 bis 12, **dadurch gekennzeichnet, daß** der Klebharzanteil, bezogen auf die Haftkleberschicht oder Wirkstoffmatrix, weniger als 50 Gew.-%, besonders bevorzugt 39 bis 49 Gew.-% beträgt.

14. Haftkleberschicht, Wirkstoffmatrix oder transdermales therapeutisches System nach einem der Ansprüche 8 bis 13, **dadurch gekennzeichnet, daß** sie einen oder mehrere penetrationsfördernde Stoffe in einem Anteil von 0,1 bis 30 Gew.-%, bezogen auf die Haftkleberschicht oder Wirkstoffmatrix, enthält, wobei der/die penetrationsfördernde(n) Stoffe vorzugsweise aus der Gruppe ausgewählt ist/sind, die gesättigte oder ungesättigte Fettsäuren, geradkettige oder verzweigte Fettalkohole sowie deren Ester, mehrwertige aliphatische Alkohole, Polyethylenglykole, Sorbitanfettsäureester sowie deren durch Ethoxylierung erhältlichen Derivate oder Fettalkohol-Ethoxylate, und monocyclische Monoterpene enthält.

15. Transdermales therapeutisches System nach einem der Ansprüche 9 bis 14, **dadurch gekennzeichnet, daß** die Wirkstoffmatrix ein oder mehrere Steroidhormone, vorzugsweise Estrogen(e) oder Estrogen(e) in Kombination mit mindestens einem Gestagen, enthält, wobei die gesamte Konzentration dieser Hormone 0,1 bis 15 Gew.-% beträgt, bezogen auf die Wirkstoffmatrix, und wobei - im Falle einer Estrogen-Gestagen-Kombination - Estrogen(e) und Gestagen(e) vorzugsweise in einem molaren Verhältnis von 1:1 bis 1:10 vorliegen.

16. Transdermales therapeutisches System nach einem der Ansprüche 9 bis 15,
**dadurch gekennzeichnet, daß** die Wirkstoffmatrix einen oder mehrere hydrolyseanfällige Wirkstoffe enthält, insbesondere Wirkstoffmoleküle mit Acetylfunktion, besonders bevorzugt Acetylsalicylsäure oder Diamorphin, oder daß sie saure oder basische Arzneimittelwirkstoffe oder deren pharmazeutisch akzeptablen Salze enthält.

17. Transdermales therapeutisches System nach einem der vorangehenden Ansprüche, **dadurch gekennzeichnet, daß**
- der Wirkstoffgehalt in der Matrix 0,1 bis 50 Gew.-% beträgt;
- der Klebharz-Anteil in der Matrix 39-49 Gew.-% beträgt;
- die wirkstoffhaltige Matrix optional penetrationsfördernde Substanzen in einer Konzentration von 0,1 bis 30 Gew.-% enthält.

18. Transdermales therapeutisches System mit einer wirkstoffhaltigen Matrix und einer die Wirkstoffabgabe kontrollierenden Steuermembran, **dadurch gekennzeichnet, daß** die Steuermembran eine adhäsive Membran oder Schicht ist, die aus einer Haftklebermasse nach einem der Ansprüche 1 bis 7 hergestellt ist.

19. Verwendung einer Haftklebermasse nach einem der Ansprüche 1 bis 7 zur Herstellung von Wundschnellverbänden, Fixierpflastern, selbstklebenden Elektroden, transdermalen therapeutischen Systemen (TTS), adhäsiven Steuermembranen in membrankontrollierten TTS, oder zusätzlichen hauthaftklebenden Schichten bei nichtadhäsiven TTS.

20. Verfahren zur Herstellung einer Haftklebermasse nach einem der Ansprüche 1 bis 7, welches die folgenden Schritte umfaßt:
- Lösen eines oder mehrerer Ethylen-Vinylacetat-Copolymere (Komponente A in Anspruch 1) bei einer Temperatur von 40-75 °C in einem Benzin- und/ Propylacetat enthaltenden Gemisch, oder in einem aus der Gruppe. Benzin/Propylacetat (2:1), Benzin/Butanon (1:1), Benzin/Propylacetat/Butanon (1:1:1), Hexan/Propylacetat/ Aceton (1:1:1) ausgewählten Lösemittel gemisch, wobei die angegebenen Mischungsverhältnisse sich auf Volumenanteile beziehen;
- Einarbeiten eines oder mehrerer Klebharze (Komponente B in Anspruch 1) unter Rühren und Homogenisierung bis zum vollständigen Auflösen, bei einer Temperatur von 40-75 °C.

21. Verfahren zur Herstellung einer Haftkleberschicht oder einer Wirkstoffmatrix nach einem der Ansprüche 8 bis 18, welches die folgenden Schritte umfaßt:
- Herstellen einer Haftklebermasse nach Anspruch 20;
- Hinzufügen und Einarbeiten von Wirkstoff(en) und Penetrationsenhancern, falls vorgesehen;
- nach Homogenisierung Beschichtung der Haftklebermasse auf eine wiederablösbare Schutzschicht mittels eines Auftragswerks;
- Entfernung der Lösemittel durch Trocknen der Schicht bei einer Temperatur von 30-80 °C;
- Aufkaschieren einer Rückschicht auf die getrocknete Haftkleberschicht.

## Claims

1. Pressure sensitive adhesive mass for the production of pressure sensitive adhesive layers for medical purposes, based on ethylene-vinyl acetate copolymers, **characterized in that** said pressure sensitive adhesive mass contains
- as polymer component (A): an ethylene-vinyl acetate copolymer or a combination of at least two ethylene-vinyl acetate copolymers,
- as component (B): an adhesive resin or a combination of adhesive resins at a portion of up to 55%-wt, relative to the sum of the components (A) and (B) without active substances or other auxiliary substances, and
that the components (A) and (B) are contained in dissolved form in an organic solvent mixture which contains gasoline and propyl acetate or in a solvent mixture selected from the group of gasoline/propyl acetate (2:1), gasoline/butanone (1:1), gasoline/propyl acetate/butanone (1:1:1), hexane/propyl acetate/acetone (1:1:1), the indicated mixing ratios relating to volume content.

2. Pressure sensitive adhesive mass according to claim 1, **characterized in that** the ethylene-vinyl acetate copolymer(s) (component A) has/have a vinyl acetate content of at least 28%-wt, relative to the monomer composition.

3. Pressure sensitive adhesive mass according to claim 1 or 2, **characterized in that** the component (B) is contained at a portion of at least 25%-wt, preferably at least 30%-wt, most preferably at a portion of 45 to 55%-wt, relative to the sum of the components (A) and (B).

4. Pressure sensitive adhesive mass according to any one of claims 1 to 3, **characterized in that** the component (A) is contained at a portion of at least 15%-wt, preferably at least 25%-wt, most preferably at a portion of 46 to 55%-wt, relative to the sum of the components (A) and (B).

5. Pressure sensitive adhesive mass according to claim 1 or 2, **characterized in that** component (A) is contained at a portion of from 46 to 55%-wt, and component (B) is contained at a portion of from 45 to 55%-wt, all values relative to the sum of components (A) and (B).

6. Pressure sensitive adhesive mass according to any one of claims 1 to 5, **characterized in that** the adhesive resin(s) is/are selected from the group comprising esters of colophony and esters of hydrogenated colophony, with particular preference from the group consisting of methyl esters, glycerol esters, pentaerythritol esters, maleic acid-modified pentaerythritol esters, maleic acid-modified glycerol esters and triethylene glycol esters.

7. Pressure sensitive adhesive mass according to any one of claims 1 to 6, **characterized in that** it does not contain any plasticizers and/or cross-linking agents.

8. Active substance-containing pressure sensitive adhesive layer or adhesive matrix for active substance release, for human or veterinary medicine, **characterized in that** it is produced from a pressure sensitive adhesive mass according to any one of claims 1 to 7.

9. Transdermal therapeutic system having an active substance-containing matrix, a backing layer and a removable protective layer, **characterized in that** it has at least one active substance-containing or active substance-free layer which is/are produced from a pressure sensitive adhesive mass according to any one of claims 1 to 7.

10. Transdermal therapeutic system according to claim 9 **characterized in that** the active substance matrix is a single-phase matrix and preferably single-layered.

11. Transdermal therapeutic system according to claim 9 or 10, **characterized in that** the active substance release is controlled by the vinyl acetate content of the ethylene-vinyl acetate copolymer(s) of the active substance matrix.

12. Pressure sensitive adhesive layer, active substance matrix or transdermal therapeutic system according to any one of claims 8 to 11, **characterized in that** the active substance portion, relative to the pressure sensitive adhesive layer or the active substance matrix, is 0.1 to 50%-wt.

13. Pressure sensitive adhesive layer, active substance matrix or transdermal therapeutic system according to any one of claims 8 to 12, **characterized in that** the adhesive resin portion, relative to the pressure sensitive adhesive layer or the active substance matrix, is less than 50%-wt, with particular preference 39 to 49%-wt.

14. Pressure sensitive adhesive, active substance matrix or transdermal therapeutic system according to any one of claims 8 to 13, **characterized in that** it contains one or more penetration-enhancing substances in a portion of 0.1 to 30%-wt., relative to the pressure sensitive adhesive layer or active substance matrix, with the penetration-enhancing substance(s) preferably being selected from the group containing saturated or unsaturated fatty acids, straight-chain or branched fatty alcohols as well as their esters, polyhydric aliphatic alcohols, polyethylene glycols, sorbitan fatty acid esters as well as their derivatives or fatty alcohol ethoxylates obtainable by ethoxylation, and monocyclic monoterpenes.

15. Transdermal therapeutic system according to any one of claims 9 to 14, **characterized in that** the active substance matrix contains one or more steroid hormones, preferably estrogen(s) or estrogen(s) in combination with at least one gestagen, with the overall concentration of these hormones being 0.1 to 15%-wt, relative to the active substance matrix, and - in the case of an estrogen-gestagen combination - with estrogen(s) and gestagen(s) preferably being present in a molar ratio of from 1:1 to 1:10.

16. Transdermal therapeutic system according to any one of claims 9 to 15, **characterized in that** the active substance matrix contains one or more active substances which are subject to hydrolysis, especially active substance molecules with acetyl function, with particular preference acetylsalicylic acid or diamorphine, or that it contains acid or basic medicinal active substances or their pharmaceutically acceptable salts.

17. Transdermal therapeutic system according to any one of the preceding claims, **characterized in that**
- the active substance content in the matrix is 0.1 to 50%-wt;
- the adhesive resin portion in the matrix is 39-49%-wt;
- the active substance-containing matrix optionally contains penetration-enhancing substances at a concentration of from 0.1 to 30%-wt.

18. Transdermal therapeutic system having an active substance-containing matrix and a control membrane controlling the active substance release, **characterized in that** the control membrane is an adhesive membrane or layer which is produced from a pressure sensitive adhesive mass according to any one of claims 1 to 7.

19. Use of a pressure sensitive adhesive mass according to any one of claims 1 to 7 for the production of adhesive dressings, fixation plasters, self-adhesive electrodes, transdermal therapeutic systems (TTS), adhesive control membranes in membrane-controlled TTS, or, in the case of non-adhesive TTS, additional layers which are pressure sensitive adhesive on the skin.

20. Process for the production of a pressure sensitive adhesive mass according to any one of claims 1 to 7, said process comprising the following steps:
- dissolving one or more ethylene-vinyl acetate copolymers (component A in claim 1) at a temperature of 40-75 °C in a gasoline-containing and propyl acetate-containing mixture or in a solvent mixture selected from the group of gasoline/propyl acetate (2:1), gasoline/butanone (1:1), gasoline/propyl acetate/butanone (1:1:1), hexane/propyl acetate/acetone (1:1:1), the indicated mixing ratios relating to volume content;
- incorporating one or more adhesive resins (component B in claim 1) by stirring and homogenising until complete dissolution occurs, at a temperature of 40-75 °C;

21. Method for the production of a pressure sensitive adhesive layer or an active substance matrix according to any one of the claims 8 to 18, said process comprising the following steps:
- preparing a pressure sensitive adhesive mass according to claim 20;
- adding and incorporating active substance(s) and penetration enhancers, if provided for;
- after homogenisation, coating the pressure sensitive adhesive mass onto a detachable protective layer by means of an applicator unit;
- removing the solvents by drying the layer at a temperature of 30-80 °C;
- laminating a backing layer onto the dried pressure sensitive adhesive layer.

## Revendications

1. Masse autoadhésive pour la préparation de couches autoadhésives destinées à des fins médicales à base de copolymères d'éthylène-acétate de vinyle, **caractérisée en ce que** la masse autoadhésive contient
- comme composant polymère (A) un copolymère d'éthylène-acétate de vinyle ou une combinaison d'au moins deux copolymères d'éthylène-acétate de vinyle,
- comme composant (B) une résine adhésive ou une combinaison de résines adhésives en une proportion allant jusqu'à 55% en poids, par rapport à la somme des composants (A) et (B) sans substance active ni d'autres adjuvants et
les composants (A) et (B) sont contenus sous forme dissoute dans un mélange de solvants organiques contenant de l'essence et de l'acétate de propyle ou dans un mélange de solvants choisi dans le groupe formé par les mélanges essence/acétate de propyle (2:1), essence/butanone (1:1), essence/acétate de propyle/butanone (1:1:1), hexane/acétate de propyle/acétone (1:1:1), les rapports de mélange indiqués se rapportant aux proportions volumiques.

2. Masse autoadhésive selon la revendication 1, **caractérisée en ce que** le(s) copolymère(s) d'éthylène-acétate de vinyle (composant A) présente(nt) une teneur en acétate de vinyle d'au moins 28% en poids par rapport à la composition de monomères.

3. Masse autoadhésive selon la revendication 1 ou 2, **caractérisée en ce que** le composant (B) est contenu en une proportion d'au moins 25% en poids, de préférence d'au moins 30% en poids, le plus préférablement en une proportion de 45 à 55% en poids, par rapport à la somme des composants (A) et (B).

4. Masse autoadhésive selon l'une quelconque des revendications 1 à 3, **caractérisée en ce que** le composant (A) est contenu en une proportion d'au moins 15% en poids, de préférence d'au moins 25% en poids, le plus préférablement en une proportion de 46 à 55% en poids, par rapport à la somme des composants (A) et (B).

5. Masse autoadhésive selon la revendication 1 ou 2, **caractérisée en ce que** le composant (A) est contenu en une proportion de 46 à 55% en poids et le composant (B) en une proportion de 45 à 55% en poids, à chaque fois par rapport à la somme des composants (A) et (B).

6. Masse autoadhésive selon l'une quelconque des revendications 1 à 5, **caractérisée en ce que** la/les résine(s) adhésive(s) est/sont choisie(s) dans le groupe comprenant les esters du colophonium et les esters du colophonium hydrogéné, de manière particulièrement préférée dans le groupe comprenant les esters de méthyle, les esters de glycérol, les esters de pentaérythritol, les esters de pentaérythritol modifiés par l'acide maléique, les esters de glycérol modifiés par l'acide maléique et les esters de triéthylèneglycol.

7. Masse autoadhésive selon l'une quelconque des revendications 1 à 6, **caractérisée en ce qu'**elle ne contient pas de plastifiant et/ou de réticulant.

8. Couche autoadhésive ou matrice adhésive contenant une/des substance(s) active(s) pour la libération d'une/de substance(s) active(s) destinée à la médecine humaine ou vétérinaire, **caractérisée en ce qu'**elle est préparée à partir d'une masse autoadhésive selon l'une quelconque des revendications 1 à 7.

9. Système thérapeutique transdermique présentant une matrice contenant une/des substance(s) active(s), une couche dorsale et une couche de protection amovible, **caractérisé en ce qu'**il présente au moins une couche contenant une/des substance(s) active(s) ou sans substance active, qui est préparée à partir d'une masse autoadhésive selon l'une quelconque des revendications 1 à 7.

10. Système thérapeutique transdermique selon la revendication 9, **caractérisé en ce que** la matrice à substance(s) active(s) est monophasique et de préférence monocouche.

11. Système thérapeutique transdermique selon la revendication 9 ou 10, **caractérisé en ce que** la libération de la/des substance(s) active(s) est contrôlée par la teneur en acétate de vinyle du/des copolymère(s) d'éthylène-acétate de vinyle de la matrice à substance(s) active(s).

12. Couche autoadhésive, matrice à substance(s) active(s) ou système thérapeutique transdermique selon l'une quelconque des revendications 8 à 11, **caractérisé(e) en ce que** la proportion de substance(s) active(s), par rapport à la couche auto-adhésive ou à la matrice à substance(s) active(s) est de 0,1 à 50% en poids.

13. Couche autoadhésive, matrice à substance(s) active(s) ou système thérapeutique transdermique selon l'une quelconque des revendications 8 à 12, **caractérisé(e) en ce que** la proportion de résine adhésive, par rapport à la couche autoadhésive ou la matrice à substance(s) active(s), est inférieure à 50% en poids, de manière particulièrement préférée de 39 à 49% en poids.

14. Couche autoadhésive, matrice à substance(s) active(s) ou système thérapeutique transdermique selon l'une quelconque des revendications 8 à 13, **caractérisé(e) en ce qu**'il/elle contient une ou plusieurs substances favorisant la pénétration en une proportion de 0,1 à 30% en poids, par rapport à la couche autoadhésive ou à la matrice à substance(s) active(s), la/les substance(s) favorisant la pénétration étant de préférence choisie(s) dans le groupe contenant les acides gras saturés ou insaturés, les alcools gras linéaires ou ramifiés ainsi que leurs esters, les alcools aliphatiques polyvalents, les polyéthylèneglycols, les esters d'acides gras de sorbitane ainsi que leurs dérivés ou éthoxylates d'alcools gras pouvant être obtenus par éthoxylation et les monoterpènes monocycliques.

15. Système thérapeutique transdermique selon l'une quelconque des revendications 9 à 14, **caractérisé en ce que** la matrice à substance(s) active(s) contient une ou plusieurs hormones stéroïdiennes, de préférence un/des estrogène(s) ou un/des estrogène(s) en combinaison avec au moins un gestagène, la concentration totale de ces hormones étant de 0,1 à 15% en poids, par rapport à la matrice à substance(s) active(s) et où - dans le cas d'une combinaison estrogène-gestagène - le(s) estrogène(s) et le(s) gestagène(s) se trouvent de préférence dans un rapport molaire de 1:1 à 1:10.

16. Système thérapeutique transdermique selon l'une quelconque des revendications 9 à 15, **caractérisé en ce que** la matrice à substance(s) active(s) contient une ou plusieurs substance(s) active(s) sensible(s) à l'hydrolyse, en particulier des molécules de substance(s) active(s) avec une fonction acétyle, de manière particulièrement préférée l'acide acétylsalicylique ou la diamorphine, ou **en ce qu'**elle contient une/des substance(s) active(s) médicamenteuse(s) acide(s) ou basique(s) ou ses/leurs sels pharmaceutiquement acceptables.

17. Système thérapeutique transdermique selon l'une quelconque des revendications précédentes, **caractérisé en ce que**
- la teneur en substance(s) active(s) dans la matrice est de 0,1 à 50% en poids ;
- la proportion de résine(s) adhésive(s) dans la matrice est de 39-49% en poids ;
- la matrice contenant une/des substance(s) active(s) contient éventuellement des substances favorisant la pénétration en une concentration de 0,1 à 30% en poids.

18. Système thérapeutique transdermique présentant une matrice contenant une/des substance(s) active(s) et une membrane de contrôle, contrôlant la libération de la/des substance(s) active(s), **caractérisé en ce que** la membrane de contrôle est une membrane ou une couche adhésive réalisée à partir d'une masse autoadhésive selon l'une quelconque des revendications 1 à 7.

19. Utilisation d'une masse autoadhésive selon l'une quelconque des revendications 1 à 7 pour la préparation de pansements rapides, de sparadraps de fixation, d'électrodes autoadhésives, de systèmes thérapeutiques transdermiques (STT), de membranes de contrôle adhésives dans les STT contrôlés par des membranes ou de couches supplémentaires adhésives sur la peau dans le cas d'un STT non adhésive.

20. Procédé pour la préparation d'une masse autoadhésive selon l'une quelconque des revendications 1 à 7, qui présente les étapes suivantes :
- dissolution d'un ou de plusieurs copolymères d'éthylène-acétate de vinyle (composant A dans la revendication 1) à une température de 40-75°C dans un mélange contenant de l'essence et de l'acétate de propyle, ou dans un mélange de solvants choisi dans le groupe constitué par les mélanges essence/acétate de propyle (2:1), essence/butanone (1:1), essence/acétate de propyle/butanone (1:1:1), hexane/acétate de propyle/acétone (1:1:1), les rapports de mélange indiqués se rapportant à des proportions volumiques ;
- incorporation d'une ou de plusieurs résines adhésives (composant B dans la revendication 1) sous agitation et homogénéisation jusqu'à dissolution complète, à une température de 40-75°C.

21. Procédé pour la préparation d'une couche autoadhésive ou d'une matrice à substance(s) active(s) selon l'une quelconque des revendications 8 à 18, qui présente les étapes suivantes :
- préparation d'une masse autoadhésive selon la revendication 20 ;
- addition et incorporation de substance(s) active(s) et d'agents favorisant la pénétration, si ceux-ci sont prévus ;
- après homogénéisation, revêtement de la masse autoadhésive sur une couche de protection amovible au moyen d'un dispositif d'application ;
- élimination du solvant par séchage de la couche à une température de 30-80°C ;
- Contre-collage d'une couche dorsale sur la couche autoadhésive séchée.
